# EUROPÄISCHE PATENTSCHRIFT

(11) **EP 0 789 014 B1**
(45) Veröffentlichungstag und Bekanntmachung des Hinweises auf die Patenterteilung: **04.10.2001**
(21) Anmeldenummer: 97101436.0
(22) Anmeldetag: 30.01.1997
(51) Int. Cl.: C07C 53/10, B01J 2/04

(54) **Herstellung von im wesentlichen wasserfreiem Bariumacetat und Strontiumacetat-Hydrat**
Preparation of essentially anhydrous barium acetate and strontium acetate-hydrate
Préparation d'acétate de barium et d'acétate-hydrate de strontium au fond anhydre

(30) Priorität: 07.02.1996 DE 19604369
(43) Veröffentlichungstag der Anmeldung: 13.08.1997
(73) Patentinhaber: SOLVAY BARIUM STRONTIUM GmbH, D-30173 Hannover (DE)
(72) Erfinder: Köhler, Karl, Dr., 31199 Diekholzen (DE); Hardinghaus, Ferdinand, Dr., 53557 Bad Hönningen (DE)
(74) Vertreter: Lauer, Dieter, Dr.

(56) Entgegenhaltungen:
- EP-A- 0 199 930
- US-A- 4 451 330

## Beschreibung

Die vorliegende Erfindung bezieht sich auf die Herstellung von im wesentlichen wasserfreiem Bariumacetat und Strontiumacetat-Hydrat.

Bariumacetat und Strontiumacetat-Hydrat sind Ausgangsmaterialien in der chemischen Synthese. Die Verbindungen können auch bei der Herstellung von Katalysatoren für die direkte Zersetzung von Stickoxiden eingesetzt werden.

Die US-A 4,451,330 offenbart ein Verfahren zur Rückgewinnung von Alkalimetallsalzen von Phenolaten und Carboxylaten. Dabei wird eine wäßrige Lösung der Salze sprühgetrocknet, um feinteiliges Salz zu gewinnen. Die EP-A-0 199 930 offenbart ein Verfahren zur Herstellung anorganischer Oxide. Dabei werden nichtwäßrige Gele oder Lösungen von hydrolysierbaren Metallverbindungen mit Wasser versetzt und mindestens teilweise hydrolysiert und sprühgetrocknet. Metallacetate sind als brauchbare Ausgangsverbindungen genannt.

Die Isolierung von Bariumacetat und Strontiumacetat bzw. Strontiumacetat-Halbhydrat erfolgte bislang durch Kristallisation aus wäßriger Lösung, siehe z. B. US-A 2,459,302. Im Handbuch "Kirk Othmer, Encyclopedia of Chemical Technology" 3^{rd} Edition, Vol. 21, Seite 765, letzte Zeile, wird festgestellt, daß Strontiumacetat sich zersetzt, wenn es erhitzt wird.

Dessen ungeachtet wurde gefunden, daß im wesentlichen wasserfreies Bariumacetat und Strontiumacetat-Hydrat mit hohem Durchsatz und hoher Ausbeute aus wäßrigen Lösungen gewonnen werden können, wenn man eine wäßrige Lösung von Bariumacetat bzw. Strontiumacetat sprühtrocknet. Der Begriff "Strontiumacetat-Hydrat" bezeichnet im Rahmen der vorliegenden Erfindung ein Produkt der Formel Sr(CH₃COO)₂ • xH₂O mit 0 < x ≤ 0,5, vorzugsweise 0,1 ≤ x ≤ 0,5.

Sofern man im wesentlichen wasserfreies Bariumacetat herstellt, setzt man zweckmäßig eine bis zu 42 Gew.-%, vorzugsweise eine 35 bis 42 Gew.-% Bariumacetat enthaltende wäßrige Lösung ein.

Zur Herstellung von Strontiumacetat-Hydrat setzt man zweckmäßig eine bis zu 40 Gew.-%, vorzugsweise 20 bis 30 Gew.-%, insbesondere 20 bis 25 Gew.-% Strontiumacetat enthaltende Lösung ein.

Die Sprühtrocknungsbedingungen werden so einreguliert, daß das getrocknete Bariumacetat den Sprühtrockner mit einer Austrittstemperatur von 100 bis 120 °C verläßt, das Strontiumacetat-Hydrat mit einer Austrittstemperatur von 100 bis 120 °C den Sprühtrockner verläßt. Das sprühgetrocknete Strontiumacetat kann noch bis zu 0,5 Mol Kristallwasser pro Mol Strontiumacetat enthalten. Je höher die Austrittstemperatur, desto geringer der Kristallwassergehalt. Durch das erfindungsgemäße Verfahren wird somit bei Bariumacetat der gesamte Wassergehalt entfernt. Bei Strontiumacetat wird der Gehalt an freiem Wasser entfernt sowie je nach Temperatur und Dauer der Erhitzung auch der ganze oder ein Teil des Kristallwassergehalts, welcher maximal 0,5 Mol pro Mol Strontiumacetat beträgt.

Der Begriff "Sprühtrocknungsbedingungen" umfaßt den Durchsatz an wäßriger Lösung pro Zeiteinheit in Abhängigkeit von der Art und Temperatur des Trocknungsgases, dem Feuchtigkeitsgehalt, der Temperatur und dem Volumenstrom des Trocknungsgases, ferner die Eintrittstemperatur der Zubereitung in die verwendete Trocknungsapparatur, den prozentualen Gehalt der wäßrigen Zubereitung an dem jeweiligen Acetat und auch von der Leistungsfähigkeit der verwendeten Trocknungsapparatur. Generelle Verfahrensbedingungen können nicht angegeben werden, da sie sehr von der jeweils verwendeten Apparatur abhängen. Für den Fachmann ist es jedoch anhand orientierender Versuche leicht, die erforderlichen Verfahrensbedingungen festzulegen. Für orientierende Versuche kann man zweckmäßig von auf etwa 300 °C vorgeheizter Luft oder Inertgas (z. B. Stickstoff) als Trocknungsgas ausgehen, und man verwendet eine wäßrige Acetat-Zubereitung, deren Konzentration im mittleren Bereich liegt. Sehr gut geeignet sind Acetat-Zubereitungen, deren Konzentration an Strontiumacetat bei 25 Gew.-% bzw. bei Bariumacetat bei etwa 40 Gew.-% liegen.

Das für die Durchführung des erfindungsgemäßen Verfahrens benötigte Acetat kann beispielsweise - gewünschtenfalls auch in situ - aus Strontiumhydroxid bzw. Bariumhydroxid oder deren Hydraten und Essigsäure hergestellt werden.

Das erfindungsgemäße Verfahren gestattet die Herstellung von im wesentlichen wasserfreiem Bariumacetat bzw. Strontiumacetat-Hydrat mit hohem Durchsatz und in energiesparender Weise. Anders als bei der Kristallisation aus wäßriger Lösung wird das in der Zubereitung enthaltene Acetat praktisch quantitativ gewonnen; es fällt feinpulverig an.

Die folgenden Beispiele sollen die Erfindung weiter erläutern, ohne sie in ihrem Umfang einzuschränken.

### Beispiel 1:

### Sprühtrocknung einer Strontiumacetat-Lösung

Eine Strontiumacetat-Lösung mit einem Gehalt an etwa 25 Gew.-% Strontiumacetat wurde nach Filtration von etwaig mitgeführten Feststoffen in einen Sprühtrockner der Firma NIRO aufgegeben. Die Eintrittstemperatur der Lösung betrug 20 °C, die Austrittstemperatur des getrockneten Gutes betrug etwa 100 °C. Als Trocknungsgas fungierte durch verbranntes Erdgas aufgeheizte Luft, welche mit einer Temperatur von 300 °C in den Sprühtrockner eingeführt wurde.

Das sprühgetrocknete Strontiumacetat-Hydrat (x= ca. 0,5) war (abgesehen vom Kristallwassergehalt) im wesentlichen wasserfrei.

### Beispiel 2:

### Sprühtrocknung von Bariumacetat-Lösung

Eine Bariumacetat-Lösung mit einem Bariumacetat-Gehalt von 40 Gew.-% wurde eingesetzt. Sie wurde mit einer Eingangstemperatur von 20 °C in den Sprühtrockner eingegeben, die Temperatur des den Sprühtrockner verlassenden getrockneten Bariumacetats betrug 105 °C. Das Produkt Ba(CH₃COO)₂ war im wesentlichen wasserfrei.

## Patentansprüche

1. Verfahren zur Herstellung von im wesentlichen wasserfreiem Bariumacetat und Strontiumacetat-Hydrat der Form Sr(CH₃COO)₂ · x H₂O mit 0 < x ≤ 0,5, wobei man eine wäßrige, bis 42 Gew.-% Bariumacetat enthaltende Lösung bzw. eine wäßrige, bis 40 Gew.-% Strontiumacetat enthaltende Lösung sprühtrocknet.

2. Verfahren nach Anspruch 1, **dadurch gekennzeichnet, daß** man zur Herstellung von Bariumacetat die Sprühtrocknungs-Bedingungen derart einreguliert, **daß** das getrocknete Bariumacetat den Sprühtrockner mit einer Austrittstemperatur von 100 bis 120 °C verläßt.

3. Verfahren nach Anspruch 1, **dadurch gekennzeichnet, daß** man zur Herstellung von Strontiumacetat-Hydrat eine 20 bis 25 Gew.-% Strontiumacetat enthaltende Lösung einsetzt.

4. Verfahren nach Anspruch 1 oder 3, **dadurch gekennzeichnet, daß** man zur Herstellung von Strontiumacetat-Hydrat die Sprühtrocknungs-Bedingungen derart einreguliert, **daß** das getrocknete Strontiumacetat-Hydrat den Sprühtrockner mit einer Austrittstemperatur von 100 bis 120 °C verläßt.

## Claims

1. A process for the preparation of substantially anhydrous barium acetate and strontium acetate hydrate of the form Sr(CH₃COO)₂ x H₂O with 0 < x 0.5, in which an aqueous solution containing up to 42% by weight barium acetate or an aqueous solution containing up to 40% by weight strontium acetate, respectively, is spray-dried.

2. A process according to Claim 1, **characterised in that** in order to prepare barium acetate the spray-drying conditions are adjusted such that the dried barium acetate leaves the spray-dryer at an exit temperature of 100 to 120°C.

3. A process according to Claim 1, **characterised in that** in order to prepare strontium acetate hydrate a solution containing 20 to 25% by weight strontium acetate is used.

4. A process according to Claim 1 or 3, **characterised in that** in order to prepare strontium acetate hydrate the spray-drying conditions are adjusted such that the dried strontium acetate hydrate leaves the spray-dryer at an exit temperature of 100 to 120°C.

## Revendications

1. Procédé de préparation d'acétate de baryum essentiellement anhydre et d'acétate de strontium hydraté de forme Sr(CH₃COO)₂.xH₂O, avec 0 < x ≤ 0,5, dans lequel on sèche par atomisation une solution aqueuse contenant jusqu'à 42 % en poids d'acétate de baryum ou selon les cas une solution aqueuse contenant jusqu'à 40 % en poids d'acétate de strontium.

2. Procédé selon la revendication 1, **caractérisé en ce que** l'on régule, pour préparer l'acétate de baryum, les conditions de séchage par atomisation de manière à ce que l'acétate de baryum séché quitte le séchoir atomiseur à une température de sortie de 100 à 120 °C.

3. Procédé selon la revendication 1, **caractérisé en ce que** l'on utilise, pour préparer l'acétate de strontium hydraté, une solution contenant 20 à 25 % en poids d'acétate de strontium.

4. Procédé selon la revendication 1 ou 3, **caractérisé en ce que** l'on régule, pour préparer l'acétate de strontium hydraté, les conditions de séchage par atomisation de manière à ce que l'acétate de strontium hydraté séché quitte le séchoir atomiseur à une température de sortie de 100 à 120 °C.
